# EUROPÄISCHE PATENTANMELDUNG

(11) **EP 4 552 529 A1**
(43) Veröffentlichungstag der Anmeldung: **14.05.2025**
(21) Anmeldenummer: 24173512.5
(22) Anmeldetag: 30.04.2024
(51) Int. Cl.: A45D 44/22, A61B 17/54, A46B 1/00

(54) **PEELING-GERÄT**

(71) Anmelder: Majesty Cosmetic GmbH, 4665 Oftringen (CH)
(72) Erfinder: VITALI, Adriana, 4852 Rothrist (CH)
(74) Vertreter: Keller Schneider Patent- und Markenanwälte AG

(57) **Zusammenfassung**

Die Erfindung betrifft ein Peeling-Gerät, insbesondere einen Peeling-Pen, insbesondere für Gesichtsbehandlungen, umfassend
a) einen Griffkörper, insbesondere einen stabförmigen Griffkörper, wobei der Griffkörper ein erstes und ein zweites Ende aufweist, und
b) Schabelemente, insbesondere Lamellen, wobei die Schabelemente am ersten Ende des Griffkörpers als geschlossene Kurven ausgebildet und konzentrisch angeordnet sind und wobei jedes der Schabelemente jeweils eine Oberkante aufweist.

## Beschreibung

### Technisches Gebiet

Die Erfindung betrifft ein Peeling-Gerät, insbesondere einen Peeling-Pen, insbesondere für Gesichtsbehandlungen, sowie die Verwendung eines solchen Peeling-Gerätes für kosmetische Gesichtsbehandlungen, insbesondere zur Entfernung von abgestorbenen Hautzellen.

### Stand der Technik

Peeling-Geräte sind aus dem Stand der Technik bekannt. Sie werden zur Reinigung, Glättung und zur Verjüngung der Haut eingesetzt, indem sie beispielsweise abgestorbene Hautzellen entfernen, die Zellerneuerung fördern und die Hauttextur verbessern. Peeling-Geräte können mechanisch einsetzbar sein, z.B. in Form von Pads mit Massagennoppen, oder elektrisch angetrieben werden, z.B. durch Batterien oder über ein Stromversorgungskabel.

Die WO 2012/104162 A1 von L'Oréal beschreibt eine Massageeinheit umfassend einen Trägerkörper sowie auf dem Trägerkörper angebrachte Massageelemente in Form von Stiften und geschwungenen Lamellen. Die Massageeinheit kann in einen Behälter eingreifen, der bspw. ein Reinigungsprodukt beinhaltet, welches über die Massageeinheit während der Reinigung abgegeben werden kann. In einer Ausführungsform können die Massageelemente in Form von elliptischen und kreisförmigen Lamellen vorhanden sein.

Die EP 2 926 792 A1 (Swiscovital AG) befasst sich mit einer Massagebürste umfassend einen elastischen Bürstenkörper mit einer Oberseite und einer Unterseite, wobei beide Seiten unterschiedlich strukturierte Bereiche aufweisen, bspw. Rippen oder noppenförmige Massagestifte. Zudem weist die Massagebürste zwei Teilkörper mit unterschiedlichen Härtegraden auf. Die Oberseite der Bürste kann so ausgestaltet sein, dass sie sich zur Gesichtsbehandlung eignet, z.B. für eine Peeling-Behandlung.

Diese bislang bekannten Peeling-Geräte haben sich jedoch als unbefriedigend herausgestellt. Beispielsweise weisen Peeling-Geräte mit Noppen eine mangelhafte Hautreinigung auf, indem sie nicht alle Rückstände wie Schmutz, Öl oder abgestorbene Hautzellen von der Haut entfernen. Ausserdem bedarf es bei Peeling-Geräten mit Noppen eine Vielzahl dieser Noppen, um einen Reinigungseffekt zu erzielen. Durch die Reibung dieser Vielzahl an Noppen auf der Haut kann es beim Peeling zu Hautirritationen kommen.

Weiter haben sich die bekannten Peeling-Geräte als unhandlich herausgestellt, da sie der Benutzerin bei der Verwendung oft aus der Hand gleiten. Auch Peeling-Geräte, welche ein Haltemittel zur besseren Führung des Peeling-Gerätes aufweisen sind nachteilig, da diese Haltemittel oft sperrig sind und leicht beschädigt oder zerstört werden können, z.B. durch Abreissen.

Ein weiterer Nachteil der bekannten Peeling-Geräte ist, dass ihre Ausgestaltung eher für ebene Hautpartien geeignet sind, wie z.B. die Wangen oder die Stirn. Für unebene Partien wie z.B. für die Augen- und Nasenpartie sind konventionelle Peeling-Geräte deshalb eher ungeeignet.

### Darstellung der Erfindung

Aufgabe der Erfindung ist es daher, eine Lösung bereitzustellen, welche die im Stand der Technik vorhandenen Nachteile zumindest verringert. Insbesondere ist es eine Aufgabe der Erfindung, ein Peeling-Gerät, insbesondere einen Peeling-Pen, bereitzustellen, welches eine gründliche Exfoliation gewährleistet, angenehm zu bedienen, einfach zu reinigen und platzsparend aufbewahrbar ist.

Diese Aufgaben werden durch die Merkmale des unabhängigen Anspruchs 1 sowie die Verwendung des unabhängigen Anspruchs 15 gelöst.

Die Erfindung betrifft demnach in einem ersten Aspekt ein Peeling-Gerät, insbesondere einen Peeling-Pen, insbesondere für Gesichtsbehandlungen, umfassend
a) einen Griffkörper, insbesondere einen stabförmigen Griffkörper, wobei der Griffkörper ein erstes und ein zweites Ende aufweist, und
b) Schabelemente, insbesondere Lamellen, wobei die Schabelemente am ersten Ende des Griffkörpers als geschlossene Kurven ausgebildet und konzentrisch angeordnet sind und wobei jedes der Schabelemente jeweils eine Oberkante aufweist.

In einer besonderen Ausführungsform sind die Schabelemente so stufenförmig zueinander angeordnet, dass eine Oberkante eines äussersten Schabelements in einer Längsrichtung des Griffkörpers näher am zweiten Ende des Griffkörpers liegt als eine Oberkante eines innersten Schabelements.

Unter "Peeling" oder "Exfoliation" wird die Entfernung von abgestorbenen Hautzellen von der obersten Hautschicht mittels mechanischen oder chemischen Methoden verstanden. Beide Begriffe werden vorliegend als Synonyme verwendet. Es ist dem Fachmann klar, dass ein Peeling nebst abgestorbenen Hautzellen weitere Ablagerungen auf der Haut, wie z.B. Schmutz und/oder Öl, entfernen kann. Die Gesamtheit der Hautablagerungen, die durch ein Peeling entfernt werden können werden hier unter dem Begriff "Rückstände" zusammengefasst.

Insbesondere können mit dem innersten Schabelement Unreinheiten wie z.B. Pusteln (sog. Pickel) entfernt werden.

Schabelemente dienen dazu, auf der Hautoberfläche befindliche Rückstände mittels einem Abschaben zu entfernen.

Lamellen sind dünn gestaltete, flache Strukturen. Ihr Vorteil ist, dass sie die Hautoberfläche schonend massieren und reinigen können, ohne die Haut zu reizen oder zu schädigen. Lamellen sind deshalb auch für empfindliche Hauttypen geeignet. Viele Lamellen für die mechanische Gesichtsreinigung sind zudem leicht zu reinigen und zu desinfizieren, was ihre Hygiene und Wiederverwendbarkeit verbessert. Dies ist besonders wichtig, um das Risiko von Bakterienwachstum und Hautirritationen zu minimieren. Lamellen können tief in die Poren eindringen, um Rückstände gründlich zu entfernen. Weiter kann die Bewegung der Lamellen die Haut entspannen und zu einem erfrischten und belebten Hautgefühl führen.

Jedes Schabelement weist jeweils eine Oberkante auf. Während der Anwendung des Peeling-Gerätes stehen die Oberkanten zumindest teilweise in Kontakt mit der Haut.

Die als geschlossene Kurven ausgebildeten und konzentrisch angeordneten Schabelemente haben den Vorteil, dass das erste Ende des Peeling-Gerätes in unterschiedliche Richtungen über die Haut bewegt werden kann, ohne das Peeling-Gerät für die Richtungsänderung erst absetzen und wieder neu ansetzen zu müssen.

Durch die stufenförmige Anordnung der Schabelemente befinden sich nicht alle Oberkanten der Schabelemente auf gleicher Höhe. Dadurch ist es nicht nötig, dass das Peeling-Gerät in einem bestimmten Winkel, insbesondere in einem 90 ° - Winkel, an die zu exfolierende Hautstelle angesetzt wird, um einen Peeling-Effekt zu erhalten. Die stufenförmige Anordnung erlaubt nämlich, dass das Peeling-Gerät in unterschiedlichen Winkeln zur Haut gehalten und über die Haut bewegt werden kann und dennoch einen Peeling-Effekt erzielt.

Insbesondere erzielt das Peeling-Gerät einen Peeling-Effekt, wenn es in einem Winkel von 20 - 160 °, insbesondere 30 - 150 °, im Speziellen 40 - 140 °, bezogen auf die Hautoberfläche, über die Haut bewegt wird.

Zudem kann das Peeling-Gerät um die Querachse, um eine zur Querachse parallel liegende Achse, um eine Hochachse und/oder um eine zur Hochachse parallel liegende Achse des Peeling-Gerätes verschwenkt werden und dennoch einen Peeling-Effekt erzielen. Je nach Bedarf kann das Peeling-Gerät relativ zur exfolierenden Hautstelle so verschwenkt werden, dass eine optimale Exfoliation erzielt wird.

Ein weiterer Vorteil des erfindungsgemässen Peeling-Gerätes ist, dass das erste Ende durch die stufenförmige Anordnung der Schabelemente bezogen auf eine Seitenansicht des Peeling-Gerätes eine vom innersten zum äussersten Schabelement abfallende Form aufweist. Dadurch weist der Griffkörper am ersten Ende eine verjüngte Silhouette auf. Eine solche Silhouette erleichtert der Benutzerin den Zugang zu unebenen Hautpartien mit dem Peeling-Gerät, wie z.B. zur Augen - und/oder Nasenpartie und erlaubt so die Entfernung von Rückständen an weniger gut zugänglichen Stellen.

Insbesondere ist das erste Ende als Kopf ausgestaltet.

Mit einem Kopf ist eine gewölbte Erhebung gemeint. Vorzugsweise erstreckt sich die Erhebung vom zweiten Ende weg.

Im Besonderen ist der Kopf halbkugel-, kegel- oder pyramidenförmig.

Der Kopf kann durch seine gewölbte Form zur Stabilität der stufenförmigen Anordnung der Schabelemente beitragen, da die Schabelemente durch das Vorhandensein des Kopfes weniger hoch ausgeformt werden müssen. Beispielsweise kann der Kopf konvex gewölbt, insbesondere halbkugelförmig, sein, wodurch bereits eine Stufenform vorgegeben werden kann. Die Schabelemente können dann in diesem Beispiel entlang der konvex gewölbten, insbesondere halbkugelförmigen, Oberfläche angeordnet sein. Eine Basis des äussersten Schabelements kann somit in einer Längsrichtung des Griffkörpers näher am zweiten Ende liegen als eine Basis des innersten Schabelements und eine Oberkante eines äussersten Schabelements kann in einer Längsrichtung des Griffkörpers näher am zweiten Ende liegen als eine Oberkante des innersten Schabelements.

Es ist aber auch vorstellbar, dass das erste Ende nicht als Kopf ausgestaltet ist. Beispielsweise kann das erste Ende als Ebene ausgestaltet sein. Es ist dann möglich, dass die Basis für das jeweilige Schabelement in Längsrichtung des Griffkörpers gleich nah vom zweiten Ende angeordnet ist, wie die Basen der restlichen Schabelemente. Die stufenförmige Anordnung kann in diesem Fall dadurch erreicht werden, dass die Schabelemente unterschiedlich hoch ausgestaltet sind, und zwar so, dass die Oberkante des äussersten Schabelements in einer Längsrichtung des Griffkörpers näher am zweiten Ende des Griffkörpers liegt als die Oberkante des innersten Schabelements.

In einer besonders bevorzugten Ausführungsform ist das erste Ende als Ebene ausgestaltet und die Schabelemente sind so auf der Ebene angeordnet, dass die Oberkante des äussersten Schabelements in einer Längsrichtung des Griffkörpers näher am zweiten Ende des Griffkörpers liegt als die Oberkante des innersten Schabelements.

Insbesondere weisen die Schabelemente unterschiedliche Höhen auf. Damit kann eine stufenförmige Anordnung erzielt werden, insbesondere wenn das erste Ende als Ebene ausgestaltet ist.

Mit der Höhe ist der Abstand zwischen der Basis und der Oberkante des Schabelements gemeint.

Mit einer Basis ist der unterste Bereich eines Schabelements gemeint. Jedes Schabelement weist eine eigene Basis auf. Vorzugsweise liegt jede Basis eines Schabelements diametral gegenüber der Oberkante desselben Schabelements. Ein Schabelement ist an seiner Basis mit dem ersten Ende verbunden.

Im Besonderen ist der Kopf konvex gewölbt, bevorzugt im Wesentlichen halbkugelförmig.

Besonders bevorzugt sind die Schabelemente am Kopf angeordnet.

In einer weiteren bevorzugten Ausführungsform umfasst der Griffkörperein Halteelement.

Mit dem Halteelement ist ein Teil des Griffkörpers gemeint, welcher die Benutzerin mit der Hand umfasst.

Vorzugsweise erstreckt sich das Halteelement vom zweiten Ende des Griffkörpers bis zum Kopf. Insbesondere umfasst das Halteelement den Kopf und die Schabelemente nicht.

In einer bevorzugten Ausführungsform, in welcher das erste Ende als Ebene ausgestaltet ist, entspricht das Halteelement dem Griffkörper.

Insbesondere ist der Kopf mit dem Halteelement verbunden.

Die Verbindung zwischen Kopf und Halteelement kann eine form-, kraft, und/oder stoffschlüssige Verbindung sein. Beispielsweise kann die Verbindung eine Steck-, Schraub und/oder Klebeverbindung sein. Es ist aber auch denkbar, dass der Kopf und das Halteelement einstückig ausgeformt sind.

In einer bevorzugten Ausführungsform ist das Peeling-Gerät einstückig geformt.

Da auf diese Weise komplett auf Verbindungsstellen verzichtet wird, kann dadurch die Robustheit des Peeling-Geräts erhöht werden. Beispielsweise kann damit auch das Risiko vermindert werden, dass das Gerät auseinanderbricht, z.B. durch eine Krafteinwirkung. Weiter kann dadurch verhindert werden, dass Feuchtigkeit an Verbindungsstellen ins Peeling-Gerät eintritt und zu einer Beschädigung führt. Ausserdem kann damit der Herstellungsprozess vereinfacht und die Kosten tief gehalten.

In einer besonders bevorzugten Ausführungsform ist das Halteelement und der Kopf einstückig geformt.

In einer weiteren bevorzugten Ausführungsform sind die Schabelemente und der Kopf einstückig geformt.

Indem das Halteelement und der Kopf oder die Schabelemente und der Kopf als einstückige Teile geformt werden, ist es möglich, dass diese einstückig geformten Teile separat von restlichen Teilen des Peeling-Gerätes produziert werden können. Dies ist vorteilhaft, um die einstückig geformten Teile in einem anderen Material herstellen zu können als die restlichen Teile. Ein weiterer Vorteil daran ist, dass Produl<tanpassungen effizient umgesetzt werden können.

Insbesondere ist das Peeling-Gerät rotationssymmetrisch um eine Längsachse.

Dies bietet den Vorteil, dass das Peeling-Gerät nicht falsch angewendet kann, da es unerheblich ist, in welche Richtung das Peeling-Gerät über die Haut bewegt wird und/oder ob das Peeling-Gerät während der Anwendung rotiert wird.

Es ist aber auch möglich, dass das Peeling-Gerät nicht rotationssymmetrisch ist. Beispielsweise ist es möglich, dass die Oberkanten der Schabelemente auf unsymmetrische Weise angeordnet sind.

Insbesondere ist es auch möglich, dass das Halteelement nicht vollumfänglich die gleiche Form aufweist. Z.B. kann das Halteelement in Längsrichtung des Griffkörpers teilweise eben ausgestaltet sein und teilweise eine konkave Form aufweisen.

Mit der Längsachse ist vorliegend die Längsachse des Peeling-Gerätes gemeint.

In einer ganz besonders bevorzugten Ausführungsform weisen die Oberkanten der Schabelemente jeweils eine kreisrunde Form auf.

Die kreisrund geformten Schabelemente haben den Vorteil, dass das erste Ende des Peeling-Gerätes in unterschiedliche Richtungen über die Hautfläche bewegt werden kann, ohne das Peeling-Gerät für die Richtungsänderung erst absetzen und wieder neu ansetzen zu müssen. Dies ergibt sich daraus, dass durch die kreisrunde Form eine gleichmässige Struktur bereitgestellt wird, welche es erlaubt, die Exfoliation in alle Richtungen gleichmässig durchzuführen.

Ausserdem kann durch die kreisrunde Form ein rotationssymmetrische Anordnung der Schabelemente bereitgestellt werden. Dadurch spielt es für die Benutzerin keine Rolle, wie sie das Peeling-Gerät an die Hautfläche ansetzt und/oder ob sie es während der Anwendung um die Längsachse rotiert.

Es ist aber auch möglich, dass die Oberkanten eine andere Form aufweisen, insbesondere eine ovale, rechteckige, gewellte, gewinkelte und/oder gekrümmte Form.

Im Besonderen weist das Halteelement in Längsrichtung des Griffkörpers eine konkave Form auf.

Dies hat den Vorteil, dass die Benutzerin das Peeling-Gerät gut greifen und in der Hand halten kann. Ausserdem kann dadurch einem Abrutschen der Hand entgegengewirkt werden. Weiter können durch die konkave Form Materialkosten für die Produktion des Peeling-Gerätes eingespart werden.

Es ist aber auch möglich, dass das Halteelement in Längsrichtung des Griffkörpers eine geschwungene und/oder gerade Form aufweist. Insbesondere kann das Halteelement eine gewellte Form aufweisen.

In einer besonders bevorzugten Form weist das Halteelement eine gewellte Form mit mindestens 1, insbesondere mindestens 2, Senken auf. Ganz speziell weist das Halteelement eine gewellte Form mit 4 Senken auf.

Durch die gewellte Form kann die Benutzerin mindestens einen Finger in die mindestens eine Senke legen, was die Halte- und/oder Rutschsicherheit verbessern kann.

Vorzugsweise weist das Halteelement eine konkave und eine gewellte Form in Kombination auf.

In einer speziellen Ausführungsform erstreckt sich die konkave, geschwungene und/oder gerade Form über die gesamte Mantelfläche des Griffkörpers. Dies ermöglicht eine gute Grifffestigl<eit des Peeling-Gerätes. Es ist aber auch möglich, dass sie sich die konkave, geschwungene und/oder gerade Form nur über einen Teil der Mantelfläche erstreckt.

In einer besonderen Ausführungsform liegt die Basis eines äusseren Schabelements in Längsrichtung des Griffkörpers näher am zweiten Ende des Griffkörpers als die Basis eines inneren Schabelements, wobei insbesondere das äussere Schabelement einen grösseren Umfang aufweist als das innere Schabelement.

Vorzugsweise ist der grösste Abstand in Querrichtung zwischen dem inneren Schabelement und der Längsachse gleich gross oder kleiner als der kleinste Abstand zwischen der Längsachse und dem äusseren Schabelement.

Mit einem inneren Schabelement ist ein Schabelement gemeint, welches von einem äusseren Schabelement umgeben ist. Mit einem äusseren Schabelement ist ein Schabelement gemeint, das das innere Schabelement umgibt. Nach dieser Definition ist es möglich, dass ein Schabelement gleichzeitig ein inneres sowie ein äusseres Schabelement darstellt, sofern das Schabelement innerhalb sowie ausserhalb seines Umfangs jeweils ein weiteres Schabelement aufweist.

Mit dem Umfang eines Schabelements ist die Länge eines Schabelements, insbesondere an der Oberkante, gemeint. Da das Schabelement eine geschlossene Kurve ist, entspricht die Länge des Schabelements der Länge der geschlossenen Kurve.

In einer speziell bevorzugten Ausführungsform liegt die Basis des äusseren Schabelements in Längsrichtung des Griffkörpers näher am zweiten Ende des Griffkörpers als die Basis des inneren Schabelements, wobei insbesondere das äussere Schabelement einen grösseren Umfang aufweist als das innere Schabelement, und wobei das erste Ende als Kopf ausgestaltet ist.

Insbesondere liegt die Oberkante des äusseren Schabelements in Längsrichtung des Griffkörpers näher am zweiten Ende des Griffkörpers als die Oberkante des inneren Schabelements, wobei insbesondere das äussere Schabelement einen grösseren Umfang aufweist als das innere Schabelement.

In einer besonderen Ausführungsform weisen die Oberkanten eines innersten und eines zweitinnersten Schabelements den gleichen Abstand zum zweiten Ende auf, sodass beide Oberkanten gleichauf liegen und wobei insbesondere das zweitinnerste Schabelement einen grösseren Umfang aufweist als das innerste Schabelement.

Mit dem innersten Schabelement ist das der Längsachse in Querrichtung am nächsten liegende Schabelement gemeint. Das innerste Schabelement umgibt dabei kein inneres Schabelement. Das innerste Schabelement wird jedoch von mindestens einem äusseren Schabelement umgeben.

Mit dem zweitinnersten Schabelement ist das der Längsachse in Querrichtung am zweitnächsten liegende Schabelement gemeint. Das zweitinnerste Schabelement umgibt dabei genau ein inneres Schabelement, nämlich das innerste Schabelement.

In einer ganz besonders bevorzugten Ausführungsform weist der Kopf ausgehend von seinem höchsten Punkt radial nach aussen einen abfallenden Höhenverlauf auf.

Mit dem Höhenverlauf ist ein Intervall an Abständen gemeint, wobei es sich bei den Abständen um Abstände zwischen einer Grundfläche des Kopfes und einem lotrecht darüber liegenden Punkt auf einer Kopfoberfläche handelt. Mit der Grundfläche des Kopfes ist eine Fläche gemeint, mit welcher der Kopf an das Halteelement grenzt.

Mit einer Kopfoberfläche ist die Oberfläche gemeint, welche sich über den Kopf erstreckt, wobei die Grundfläche des Kopfes nicht Teil der Kopfoberfläche ist.

Der abfallende Höhenverlauf kann zur Stabilität der stufenförmigen Anordnung der Schabelemente beitragen, da die Schabelemente dadurch weniger hoch ausgeformt werden müssen. Beispielsweise kann der Kopf konvex gewölbt, insbesondere halbkugelförmig, sein, wodurch eine Stufenform bereits vorgegeben werden kann. Die Schabelemente können dann in diesem Beispiel entlang dem abfallenden Höhenverlauf auf einer Kopfoberfläche angeordnet sein. Die Basis des äusseren Schabelements kann somit in Längsrichtung des Griffkörpers näher am zweiten Ende liegen als die Basis des inneren Schabelements.

Insbesondere geht das äusserste Schabelement zumindest teilweise aus der Mantelfläche des Halteelements hervor. Insbesondere liegt ein nahtloser und/oder stufenloser Übergang zwischen der Mantelfläche des Halteelements und einer äusseren Seitenfläche des äussersten Schabelement vor. Die vorhergehend erwähnte "äussere Seitenfläche" entspricht der weiter unten eingeführten "ersten Seite" eines Schabelements, d.h. es handelt sich bei der "äusseren Seitenfläche" um eine Seite, deren Oberfläche von der Längsachse weg zeigt.

Dies hat den Vorteil, dass keine Verbindungsstellen zwischen dem äussersten Schabelement und dem Halteelement vorhanden sind. Dadurch verringert sich das Risiko einer Beschädigung des Peeling-Gerätes durch Brechen und/oder Auseinanderfallen an einer Verbindungsstelle. Zudem verbessert diese Ausführungsform die Stabilität des Peeling-Gerätes und/oder verhindert das Eindringen von Feuchtigkeit ins Innere des Peeling-Gerätes.

Insbesondere liegt jeder Querschnittsmittelpunl<t auf der Längsachse.

Mit dem Querschnittsmittelpunl<t ist der Mittelpunkt jedes Querschnittes des Peeling-Geräts gemeint. D.h. unabhängig davon, an welcher Stelle ein Querschnitt es Peeling-Gerätes erfolgt, liegt der Mittelpunkt der jeweiligen Querschnittsfläche auf der Längsachse.

In einer weiteren bevorzugten Ausführungsform ist das zweite Ende eben ausgestaltet.

Dies ist vorteilhaft, das das Peeling-Gerät mit der ebenen zweiten Fläche auf eine Oberfläche gestellt werden kann, ohne, dass das Gerät davonrollt und/oder umkippt. Dies bietet eine optimale Lösung zur Aufbewahrung des Gerätes. Zudem erweist sich diese Lösung als platzsparend, da lediglich eine Fläche in der Grösse des zweiten ebenen Endes für die Aufbewahrung benötigt wird.

Es ist aber auch denkbar, dass das zweite Ende als unebene Struktur ausgestaltet ist, z.B. in der Form von Füssen und/oder Noppen. Insbesondere sind solche Noppen nicht zur Exfoliation geeignet.

In einer weiteren Ausführungsform umfasst das zweite Ende mindestens eine Vertiefung, insbesondere mindestens eine mittig am zweiten Ende platzierte Vertiefung.

Dies bietet den Vorteil, dass das Peeling-Gerät weniger Gewicht haben kann. Zudem kann dadurch Material bei der Produktion eingespart werden. Eine weitere vorteilhafte Eigenschaft ist, dass die Vertiefung zu einem stabileren Stand des Peeling-Gerätes beitragen kann, wenn es auf eine Oberfläche abgestellt wird. Dies, da das Gerät mit der Vertiefung über eine Erhebung gestellt werden kann, z.B. auf einer Oberfläche, was ein Verrutschen des Peeling-Gerätes verringert. Zudem ist es möglich, das Peeling-Gerät je nach Länge der Vertiefung an einem Vorspruch aufzuhängen und/oder festzustecl<en.

Im Besonderen liegen sich das erste und das zweite Ende diametral gegenüber.

Dadurch kann ein Peeling-Gerät bereitgestellt werden, welches sich aufgrund seiner länglichen Form platzsparend verstauen lässt.

In einer weiteren bevorzugten Ausführungsform umfasst das Peeling-Gerät 1 - 500, insbesondere 1 - 400, im Speziellen 1 - 300, besonders bevorzugt 1 - 200, ganz besonders speziell 1 - 100, Schabelemente.

Bevorzugt umfasst das Peeling-Gerät 2 - 10, insbesondere 2 - 8, im Speziellen 3 - 7, besonders bevorzugt 6, Schabelemente.

Im Speziellen umfasst das Peeling-Gerät mehr als 4 Schabelemente, insbesondere mehr als 5 Schabelemente.

Es hat sich herausgestellt, dass die oben beschriebene Anzahl an Schabelementen eine äusserst schonende und dennoch effiziente Exfoliation ermöglichen können. Zudem erweist sich eine Reinigung als besonders einfach.

Dies hat den Vorteil, dass eine äusserst effiziente und dennoch schonende Exfoliation ermöglicht werden kann. Insbesondere können auf der Haut abgelagerte Rückstände erfolgreich entfernt werden.

Insbesondere weisen die Schabelemente eine Dicke von 0.05 - 6 mm, insbesondere 0.07 - 5.5 mm, im Speziellen 0.08 - 5.2 mm, besonders bevorzugt 0.1 - 5 mm, auf.

In einer besonders bevorzugten Ausführungsform weisen die Schabelemente eine Dicke von 0.05 - 1 mm, insbesondere 0.07 - 0.9 mm, im Speziellen 0.08 - 0.8 mm, besonders bevorzugt 0.1 - 0.7 mm, auf.

Mit der Dicke ist eine querschnittliche Ausdehnung des Schabelements gemeint, d.h. der Abstand zwischen einer ersten Seite des Schabelements zu einer gegenüberliegenden zweiten Seite des Schabelements. Mit der ersten Seite eines Schabelements ist diejenige Seite gemeint, deren Oberfläche von der Längsachse weg zeigt. Mit der zweiten Seite des Schabelements ist diejenige Seite gemeint, deren Oberfläche in Richtung der Längsachse zeigt.

Durch diese Dicke können stabile Schabelemente bereitgestellt werden, die eine lange Lebensdauer aufweisen. Zudem können die Schabelemente dadurch den Kräften standhalten, die während der Anwendung auf sie wirken. Insbesondere ist die oben erwähnt Dicke vorteilhaft, da die Schabelemente genug starr sind, um erfolgreich eine Exfoliation durchzuführen und gleichzeitig genug biegsam, sodass die Exfoliation hautschonend durchgeführt werden kann.

Besonders bevorzugt weisen die Schabelemente eine Länge von 1 - 15 cm, insbesondere 1 - 12 cm, im Speziellen 1 - 10 cm, besonders bevorzugt 1 - 8 cm, ganz besonders speziell 1 - 6 cm, ausserordentlich bevorzugt 1 - 5 cm, auf.

Durch die oben erwähnten Längen können Schabelemente bereitgestellt werden, die einen optimalen Umfang bereitstellen, um auf gründliche Weise Rückstände entfernen. Weiter kann so viel Hautfläche auf einmal bearbeitet werden, sodass die Exfoliation schnell durchgeführt werden kann. Zudem trägt diese Länge zu einem handlichen Peeling-Gerät bei, insbesondere kann das Peeling-Gerät in einer konventionellen Handtasche transportiert werden.

Mit der Länge des Schabelements ist der Umfang des Schabelements gemeint. Da das Schabelement eine geschlossene Kurve ist, entspricht die Länge des Schabelements der Länge der geschlossenen Kurve.

Insbesondere weisen die Schabelemente eine Höhe von 1 - 20 mm, insbesondere 1 - 15 mm, im Speziellen 1 - 10 mm, besonders bevorzugt 1 - 5 mm, auf.

Ein Vorteil daran ist, dass das Peeling-Gerät dadurch eine sanfte und dennoch gründliche Exfoliation bereitstellen kann. Weiter tragen diese Höhen zu einem handlichen Peeling-Gerät bei, insbesondere kann das Peeling-Gerät in einer konventionellen Handtasche transportiert werden. Zudem braucht das Peeling-Gerät dadurch nicht viel Platz für die Aufbewahrung.

Mit der Höhe ist der Abstand zwischen der Basis und der Oberkante des Schabelements gemeint.

In einer weiteren Ausführungsform ist es möglich, dass mindestens zwei, insbesondere mindestens 4, im Speziellen mindestens 6, besonders bevorzugt mindestens 8, Schabelemente unterschiedliche Höhen aufweisen.

In einer weiteren Ausführungsform ist es möglich, dass mindestens 20, insbesondere mindestens 50, im Speziellen mindestens 100, besonders bevorzugt mindestens 200, ganz speziell bevorzugt mindestens 400, Schabelemente unterschiedliche Höhen aufweisen.

Insbesondere sind die Schabelemente äquidistant zueinander angeordnet. Dadurch kann ein gleichmässiger Peeling-Effekt erzielt werden.

Es ist aber auch möglich, dass die Schabelemente nicht-äquidistant angeordnet sind.

In einer besonderen Ausführungsform sind die Schabelemente jeweils in einem Abstand von 0.5 mm - 1 cm, insbesondere 1 - 6 mm, im Speziellen 2 - 4 mm, ausserordentlich bevorzugt genau 3 mm, voneinander angeordnet.

Im Speziellen beträgt das Verhältnis zwischen dem Umfang und der Höhe der eines Schabelements zwischen 5:1 und 25:1, insbesondere zwischen 7:1 und 23:1, im Speziellen 10:1 und 21:1, ganz besonders bevorzugt zwischen 14:1 und 19:1.

Insbesondere beträgt die Gesamtlänge des Peeling-Gerätes 3 - 15 cm, insbesondere 4 - 12 cm, im Speziellen 5 - 10 cm, besonders bevorzugt 6 - 9 cm.

Diese Länge hat den Vorteil, dass das Peeling-Gerät handlich ist und überall hin mitgenommen werden kann. Beispielsweise passt das Gerät in eine Handtasche und/oder lässt sich unkompliziert zu Hause in einem Spiegelschranl< aufbewahren. Zudem benötigt das Peeling-Gerät wenig Platz in der Spülmaschine.

Mit der Gesamtlänge des Peeling-Gerätes ist der Abstand zwischen zwei in Längsrichtung voneinander am weitesten entfernten Punkten auf dem Peeling-Gerät gemeint.

In einer besonders bevorzugten Ausführungsform beträgt das Verhältnis zwischen einer Länge des Halteelements und einem Durchmesser des Halteelementes zwischen 1:1 und 5:1, insbesondere zwischen 2:1 und 4:1, im Speziellen zwischen 2.5:1 und 3.5:1, besonders bevorzugt 3:1.

In einer weiteren bevorzugten Ausführungsform sind zwischen zwei benachbarten Schabelementen Rillen ausgebildet.

Ein Vorteil davon ist, dass die von der Haut exfolierten Rückstände während der Anwendung in die Rillen fallen und so aufgefangen werden können. Das Risiko, dass Rückstände auf den Boden, die Kleidung der Benutzerin oder auf andere umliegende Gegenstände fällt, wird dadurch reduziert.

Im Besonderen liegt jede der Oberkanten in jeweils einer Ebene und die Normalen der Ebenen verlaufen entlang und/oder parallel zur Längsachse.

Damit kann ein gleichmässiges Peeling-Ergebnis erzielt werden, da die Schabelemente gleichmässig angeordnet sind. Weiter vereinfacht sich dadurch die Reinigung des Peeling-Gerätes.

In einer bevorzugten Ausführungsform sind alle Ebenen planparallel zueinander angeordnet.

Insbesondere sind die Schabelemente in Längsrichtung des Griffkörpers koaxial angeordnet.

In einer weiteren bevorzugten Ausführungsform sind alle Schabelemente in Richtung der Längsachse geneigt.

In einer besonders bevorzugten Ausführungsform sind die Schabelemente von der Längsachse weggeneigt.

Es ist aber auch möglich, dass mindestens ein Schabelement in Richtung der Längsachse geneigt ist und mindestens ein Schabelement von der Längsachse weggeneigt ist.

Insbesondere weist mindestens eine Rille, insbesondere alle Rillen, einen gekrümmten und/oder gewinkelten Rillenboden auf.

Im Besonderen weisen die Rillen jeweils einen Rillenboden auf.

In einer weiteren bevorzugten Ausführungsform bildet die Oberfläche des ersten Endes den Rillenboden.

Ein Vorteil dieser Ausführungsformen ist, dass Rückstände, die während der Anwendung des Peeling-Gerätes in die Rillen fallen, bei einer Reinigung des Gerätes einfach wieder entfernt werden können. Insbesondere kann eine einfache Reinigung des Peeling-Geräts erzielt werden, wenn der Rillenboden gekrümmt und/oder gewinkelt ist, da die Rückstände unkomplizierter aus den Rillen entfernt werden können.

Besonders bevorzugt weist der Griffl<öper über seine gesamte Länge keinen konstanten oder einen variablen Querschnitt auf.

Dies kann vorteilhaft sein, wenn das Peeling-Gerät möglichst ergonomisch gestaltet sein soll. Es ist aber auch möglich, dass das Gerät einen konstanten Querschnitt aufweist.

Mit einem nicht konstanten oder variablen Querschnitt ist gemeint, dass abhängig davon, an welcher Stelle entlang der Längsachse man sich befindet, der Querschnitt des Peeling-Gerätes unterschiedlich ausfallen kann. Entsprechend ist mit einem konstanten Querschnitt gemeint, dass unabhängig davon, an welcher Stelle entlang der Längsachse man sich befindet, der Querschnitt des Peeling-Gerätes an jeder Stelle gleich ist.

Im einer besonderen Ausführungsform weist das Peeling-Gerät an seiner breitesten Stelle einen Durchmesser von 0.5 - 10 cm, insbesondere 1 - 8 cm, im Speziellen 1.5 - 6 cm, auf.

Im Besonderen weist das Peeling-Gerät an seiner breitesten Stelle einen Durchmesser von 0.5 - 5 cm, insbesondere 1 - 4 cm, im Speziellen 1.5 - 3 cm, auf.

Insbesondere weist das Peeling-Gerät an seiner schmalsten Stelle einen Durchmesser von 0.5 - 3.5 cm, insbesondere 1 - 3 cm, im Speziellen 1.5 - 2.5 cm, auf.

Insbesondere umfasst das Peeling-Gerät keinen Antrieb, im Speziellen keinen elektrischen Antrieb. Ebenso verfügt das Peeling-Gerät bevorzugt über keine Batterie, keine Batterieaufnahme und/oder keinen Stromversorgungsanschluss.

Im Speziellen weist das Peeling-Gerät keine Noppen auf.

Dies trägt dazu bei, dass das Peeling-Gerät unabhängig von einer Energiequelle funktionieren kann. Das ist besonders vorteilhaft, da das Gerät dadurch überall angewendet werden kann. Zudem ist das Gerät umweltfreundlich, da es keinerlei Energie benötigt.

In einer bevorzugten Ausführungsform umfasst oder besteht das Peeling-Gerät AcrylnitrilButadien-Styrol, Polyvinylchlorid (PVC), Polyurethan und/oder Silikon.

Diese Material haben sich als äusserst praktisch herausgestellt, da sie dem Peeling-Gerät eine angenehme Haptik verleihen. Ein weiterer Vorteil ist, dass das Gerät dadurch spülmaschinenfest ist, was eine einfache Reinigung ermöglicht. Ein Vorteil von AcrylnitrilButadien-Styrol ist, dass dieses Material feuchtigkeitsabweisend und schmutzabweisend ist und dass es weitgehend resistent gegen Fette und Öle, gegen elektrostatische Aufladung und Temperaturschwankungen ist. PVC hat die vorteilhaften Eigenschaften, dass es unempfindlich gegenüber Temperatur und Wasser ist. Zudem ist es sehr witterungsbeständig, langlebig und lässt sich leicht einfärben. Die Vorteile von Polyurethan sind, dass das Material eine hohe mechanische Festigkeit, eine Beständigkeit gegen Temperatur sowie Öle und Fette aufweist, dass es verschleissfest ist und dass es gute Dämpfungswerte aufweist. Silikon ist vorteilhaft, da es chemisch inert, langlebig, widerstandsfähig gegen UV- und Ozon-Strahlung, ungiftig, wiederverwendbar und leicht recycelbar ist.

Im Speziellen umfasst oder besteht das Peeling-Gerät aus Polyurethan und weist eine SHORE-Härte von 30 - 90, insbesondere von 30, 35, 40, 45, 50, 55, 60, 65, 70, 75, 80, 85 oder 90, auf.

Die SHORE-Härte wird insbesondere nach der SHORE Härte-Skala A gemessen.

Im Speziellen wird die SHORE-Härte nach dem Prüfverfahren ISO 868-85 geprüft.

Im Speziellen besteht das Peeling-Gerät aus einem einzigen Material.

Dies erleichtert und beschleunigt die Herstellung des Peeling-Gerätes, da nur auf ein Material zurückgegriffen werden muss. Zudem wir das Recycling vereinfacht, da nur Sortenreine Kunststoffe zurückgewonnen werden müssen.

Insbesondere ist das Peeling-Gerät im Spritzgussverfahren und/oder im 3D-Drucl< hergestellt.

In einem zweiten Aspekt betrifft die Erfindung eine Verwendung eines Peeling-Gerätes wie vorhergehend beschrieben für kosmetische Gesichtsbehandlungen, insbesondere zur Entfernung von abgestorbenen Hautzellen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine schematische Perspektivansicht eines erfindungsgemässen Peeling-Gerätes
- Fig. 2: Ein Ausschnitt eines ersten Endes mit Schabelementen des in Fig. 1 gezeigten Peeling-Gerätes
- Fig. 3: Eine Draufsicht auf das erste Ende und die Schabelemente des Peeling-Gerätes aus Fig. 2
- Fig. 4: Eine Draufsicht auf das erste Ende und die Schabelemente einer Ausführungsform des erfindungsgemässen Peeling-Gerätes
- Fig. 5: Eine Draufsicht auf einen Längsschnitt entlang der Schnittlinie A - A' des in Fig. 1 gezeigten Peeling-Gerätes

Grundsätzlich sind in den Figuren gleiche Teile mit gleichen Bezugszeichen versehen.

### Wege zur Ausführung der Erfindung

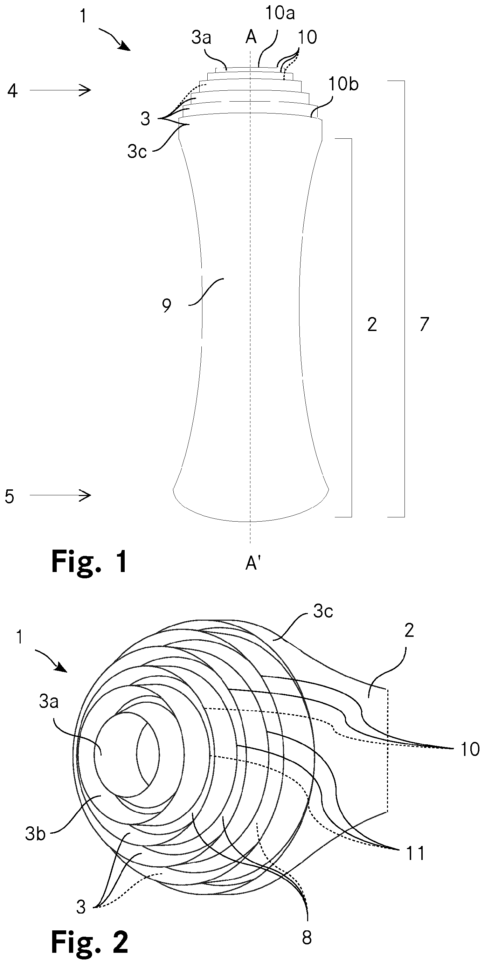

**Fig. 1** zeigt eine schematische Perspektivansicht eines erfindungsgemässen Peeling-Gerätes 1 mit einem Griffkörper7, wobei der Griffkörper7 eine Halteelement 2 und einen Kopf (nicht gezeigt) umfasst. Der Griffl<öper 7 weist ein erstes Ende 4 und ein zweites Ende 5 auf. Am ersten Ende 4 sind Schabelemente 3 stufenförmig am Kopf (nicht gezeigt) angeordnet. Weiter weist das Halteelement 2 eine konkave Form auf. Ein äusserstes Schabelement 3c geht aus der Mantelfläche 9 des Halteelements 2 hervor. Eine Oberkante 10b des äussersten Schabelements 3c liegt in einer Längsrichtung des Griffl<öpers 7 näher am zweiten Ende 5 des Griffkörpers 7 als eine Oberkante 10a eines innersten Schabelements 3a. Die konkave Form des Halteelements 2 erstreckt sich vollumfänglich um das Halteelement 4. Das erste Ende 4 liegt dem zweiten Ende 5 diametral gegenüber.

**Fig. 2** zeigt einen Ausschnitt des ersten Endes 4 und der Schabelemente 3 des in Fig. 1 gezeigten Peeling-Gerätes 1. Zwischen zwei Schabelementen 3 ist jeweils eine Rille 8 vorhanden. Der Boden jeder Rille 8 wird durch die Oberfläche des ersten Endes 4 geformt. Die Schabelemente 10 weisen jeweils eine Basis 11 auf. Die Basis 11 liegt der jeweiligen Oberkante der Schabelemente diametral gegenüber und bildet das untere Ende des Schabelements 10. Die Basen 11 sind auf der Oberfläche des Kopfs 6 angeordnet. Das Halteelement ist nur teilweise gezeigt, was durch eine gestrichelte langgezogene Linie angedeutet ist.

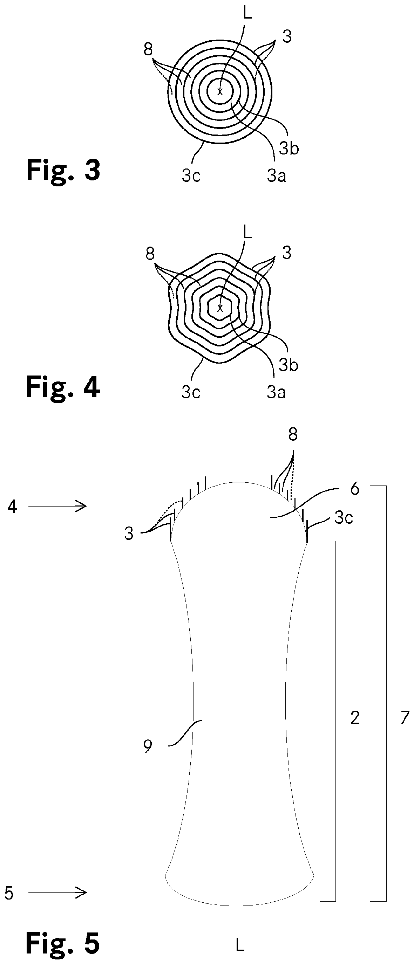

**Fig. 3** zeigt eine Draufsicht auf das erste Ende 4 und die Schabelemente 3 des in Fig. 2 gezeigten Peeling-Gerätes 1. Die Schabelemente 3 sind geschlossene Kurven und haben eine kreisrunde Form. Ausserdem sind sie konzentrisch angeordnet. Durch den gemeinsamen Mittelpunkt x der konzentrisch angeordneten Schabelemente 3 verläuft die Längsachse L. Jeweils zwischen zwei Schabelementen 3 verläuft eine Rille 8. Das innerste Schabelement 3a liegt in Querrichtung am nächsten an der Längsachse L. Das zweitinnerste Schabelement 3b liegt in Querrichtung am zweitnächsten an der Längsachse L und umgibt das innerste Schabelement 3a. Das äusserste Schabelement 3c liegt in Querrichtung am weitesten weg von der Längsachse L.

**Fig. 4** zeigt eine Draufsicht auf das erste Ende 4 und die Schabelemente 3 einer anderen Ausführungsform des erfindungsgemässen Peeling-Gerätes 1. Der Aufbau der Fig. 4 ist analog zu der Fig. 3 mit dem einzigen Unterschied, dass die Oberkanten 10 der Schabelemente 3 nicht kreisrund, sondern gewellt sind.

**Fig. 5** zeigt eine Draufsicht auf einen Längsschnitt entlang der Schnittlinie A - A' des in Fig. 1 gezeigten Peeling-Gerätes 1. Die Schabelemente 3 sind entlang des Kopfes 6 stufenförmig ansteigend zur Längsachse L hin angeordnet beginnend ab dem äussersten Schabelement 3c. Zwischen jeweils 2 Schabelementen 3 befinden sich Rillen 8. Die Schabelemente 3 sind parallel zur Längsachse L ausgerichtet.

Die Figuren 1 - 5 zeigen lediglich mögliche und nicht abschliessende Ausführungsbeispiele des Erfindungsgegenstandes, wobei dessen Merkmale entsprechend jeweiliger Anforderungen beliebig verändert, miteinander kombiniert und/oder weggelassen werden können.

Beispielsweise kann das Halteelement 2 in den Fig. 1 und 5 eine andere Form aufweisen, insbesondere eine geschwungene, gerade und/oder gewellte Form. Es ist auch möglich, dass die konkave Form nicht vollumfänglich um das Halteelement 2 verläuft, sondern nur teilweise. Ausserdem ist es denkbar, dass die Längsachse L nicht geradlinig verläuft, sondern z.B. gekrümmt. Entsprechend ist es möglich, dass das Peeling-Gerät anders als stabförmig geformt ist, z.B. gekrümmt.

Weiter ist es möglich, dass die Oberkanten 10 der Schabelemente 3 in den Fig. 1 bis 5 in Längsrichtung des Griffkörpers nicht in einer konstanten Höhe verlaufen. Es kann z.B. sein, dass die Oberkanten 10 oder zumindest eine Oberkante 10 zumindest teilweise geschwungen, gekrümmt und/oder gewellt verlaufen.

In Bezug auf die Fig. 3 und 4 ist es zudem denkbar, dass die Oberkanten 10 der Schabelemente 3 eine andere Form aufweisen, z.B. eine ovale, rechteckige, gewellte, gewinkelte und/oder gekrümmte Form.

Die Schabelemente, wie sie in den Fig. 1 - 5 gezeigt sind, können auch geneigt verlaufen, z.B. in eine Richtung zur Längsachse L hin oder in eine Richtung von der Längsachse L weg. Dabei kann mindestens ein Schabelement 3, insbesondere alle Schabelemente 3, entsprechend geneigt sein.

## Patentansprüche

1. Peeling-Gerät, insbesondere Peeling-Pen, insbesondere für Gesichtsbehandlungen, umfassend
a) einen Griffkörper, insbesondere einen stabförmigen Griffkörper, wobei der Griffkörper ein erstes und ein zweites Ende aufweist, und
b) Schabelemente, insbesondere Lamellen, wobei die Schabelemente am ersten Ende des Griffkörpers als geschlossene Kurven ausgebildet und konzentrisch angeordnet sind und wobei jedes der Schabelemente jeweils eine Oberkante aufweist.

2. Peeling-Gerät nach Anspruch 1, wobei die Schabelemente so stufenförmig zueinander angeordnet sind, dass eine Oberkante eines äussersten Schabelements in einer Längsrichtung des Griffkörpers näher am zweiten Ende des Griffkörpers liegt als eine Oberkante eines innersten Schabelements.

3. Peeling-Gerät nach einem der vorangehenden Ansprüche, wobei das erste Ende als Kopf ausgestaltet ist.

4. Peeling-Gerät nach Anspruch 3, wobei der Kopf konvex gewölbt, bevorzugt im Wesentlichen halbkugelförmig, ist.

5. Peeling-Gerät nach einem der vorangehenden Ansprüche, wobei der Griffkörperein Halteelement umfasst.

6. Peeling-Gerät nach einem der vorangehenden Ansprüche, wobei das Peeling-Gerät einstückig geformt ist.

7. Peeling-Gerät nach einem der vorangehenden Ansprüche, wobei das Peeling-Gerät rotationssymmetrisch um eine Längsachse ist.

8. Peeling-Gerät nach einem der vorangehenden Ansprüche, wobei die Oberkanten der Schabelemente jeweils eine kreisrunde Form aufweisen.

9. Peeling-Gerät nach einem der Ansprüche 5 - 8, wobei das Halteelement in Längsrichtung des Griffkörpers eine konkave Form aufweist.

10. Peeling-Gerät nach einem der vorangehenden Ansprüche, wobei die Oberkante eines äusseren Schabelements in Längsrichtung des Griffkörpers näher am zweiten Ende des Griffkörpers liegt als die Oberkante eines inneren Schabelements, wobei insbesondere das äussere Schabelement einen grösseren Umfang aufweist als das innere Schabelement.

11. Peeling-Gerät nach einem der Ansprüche 3 - 10, wobei der Kopf ausgehend von seinem höchsten Punkt radial nach aussen einen abfallenden Höhenverlauf aufweist.

12. Peeling-Gerät nach einem der vorangehenden Ansprüche, wobei das Peeling-Gerät 1 - 500, insbesondere 1 - 400, im Speziellen 1 - 300, besonders bevorzugt 1 - 200, ganz besonders speziell 1 - 100, Schabelemente umfasst.

13. Peeling-Gerät nach einem der vorangehenden Ansprüche, wobei das Peeling-Gerät mehr als 4 Schabelemente, insbesondere mehr als 5 Schabelemente, umfasst.

14. Peeling-Gerät nach einem der vorangehenden Ansprüche, wobei die Schabelemente eine Dicke von 0.05 - 6 mm, insbesondere 0.07 - 5.5 mm, im Speziellen 0.08 - 5.2 mm, besonders bevorzugt 0.1 - 5 mm, aufweisen und/oder wobei die Schabelemente eine Länge von 1 - 15 cm, insbesondere 1 - 12 cm, im Speziellen 1 - 10 cm, besonders bevorzugt 1 - 8 cm, ganz besonders speziell 1 - 6 cm, ausserordentlich bevorzugt 1 - 5 cm, aufweisen.

15. Peeling-Gerät nach einem der vorangehenden Ansprüche, wobei die Schabelemente eine Höhe von 1 - 20 mm, insbesondere 1 - 15 mm, im Speziellen 1 - 10 mm, besonders bevorzugt 1 - 5 mm, aufweisen.

16. Verwendung eines Peeling-Gerätes nach einem der vorangehenden Ansprüchen für kosmetische Gesichtsbehandlungen, insbesondere zur Entfernung von abgestorbenen Hautzellen.
